# EUROPEAN PATENT SPECIFICATION

(11) **EP 0 377 439 B1**
(45) Date of publication and mention of the grant of the patent: **13.09.1995**
(21) Application number: 90100032.3
(22) Date of filing: 02.01.1990
(51) Int. Cl.: A61K 9/52, A61K 9/20, A61K 31/60

(54) **Controlled-release, low-dose aspirin**
Niedrigdosiertes Aspirin mit gesteuerter Freigabe
Aspirine à faible dose et à libération contrôlée

(30) Priority: 03.01.1989 US 293832; 24.10.1989 US 426620
(43) Date of publication of application: 11.07.1990
(73) Proprietor: STERLING WINTHROP INC., New York, NY 10016 (US)
(72) Inventor: Frisbee, Steven Edgar, Slingerlands New York 12159 (US); Fitzgerald, Garret Adare, Nashville Tennessee 37215 (US); Charman, William Neil, Parkville Victoria 3052 (AU)
(74) Representative: Baillie, Iain Cameron

(56) References cited:
- EP-A- 0 213 083
- US-A- 4 866 046

## Description

This invention relates to the use of a coating formulation for coating aspirin granules so as to release aspirin at a rate of about 5 to 15 mg/hr, particularly for a dosage form of about 40 to 100 mg of aspirin, and to the use of coated granules or tablets made therefrom for making a medicament for the treatment or prevention of vascular occlusive diseases in humans.

The invention further relates to a process for coating aspirin granules that results in coated granules that may be compressed into tablets and that release aspirin with approximately zero-order kinetics over a period extending five to eight hours. The invention also relates to rapidly disintegrating tablets comprising said coated aspirin granules and a process for the manufacture of said tablets. In a more specific aspect the invention relates to processes for and products from the coating of aspirin granules with a neutral, insoluble but permeable, elastic film and the fashioning of tablets from said coated granules by compressing with disintegrants, antiadherents, lubricants and preferentially-crushable granules.

It is known in the art that aspirin significantly decreases platelet adhesiveness by acetylating cyclooxygenase in platelets. This biochemical action manifests itself in statistically significant protection from vascular occlusive events among patients given oral aspirin. It is also known that, at the doses given in most clinical trials, aspirin inhibits endothelial cyclooxygenase. Because the effects in these two tissues are believed to antagonize one another, there has been a search for a dose, a delivery rate, and a dosage form of aspirin that might be effective in suppressing the production of thromboxane B₂(TX) by platelets without suppressing the production of prostacyclin (PC) by endothelial cells.

More recently Reilly and FitzGerald [Clinical Research 32, 320A(1984)] have suggested that if hepatic extraction were virtually complete with low doses of aspirin, repeated administration of low doses might permit cumulative presystemic inhibition of TX while protecting endothelial cyclooxygenase from exposure to aspirin. They reported that 1 mg of aspirin given every 30 minutes for 10 hours to human volunteers resulted in a decrease in serum TX by 66% at 10 hours while urinary PC was unaltered. Similarly, Jakubowski et al. [J. Lab. Clin. Med. 108, 616-621(1986)] reported that granular, enteric-coated aspirin, when given three times a day in 27 mg doses to provide a "continuous, low-dose, antiplatelet regimen" resulted in 96% inhibition of serum TX generation without detectable levels of aspirin in the systemic circulation of human volunteers. Formal clinical trials with low-dose (50 to 100 mg), slow-release aspirin to treat or prevent transient ischemic attacks, myocardial infarction and related vascular occlusive events were suggested by Bochner and Lloyd [Clin. Sci. 71, 625-631(1986)].

U.S. Patent 4,866,046 describes a tablet containing from about 30 mg. to about 190 mg. of aspirin capable of rapid dissolution or disintegration in the oral, preferably sublingual, cavity permitting rapid absorption of the aspirin from the saliva into the blood system. The reference does not describe coated aspirin granules, zero-order release kinetics, release rates, copolymer of ethyl acrylate and methyl methacrylate or hydroxypropylmethylcellulose as coating material for aspirin or aqueous spray coating of aspirin and therefore does not suggest the presently described and claimed invention.

Until our discovery, no low-dose, controlled-release formulation for aspirin was known and the precise combination of dose and delivery rate to produce the optimum differentiation between inhibition of thromboxane and inhibition of prostacyclin was likewise unknown. Methods and formulations in the literature have suggested ways that one might approach the problem of dosage form, but none of them addresses the five parallel requirements that we believe are necessary for large scale production and ultimate commercial distribution to the patient population: (1) A dosage form must provide a controlled, linear, near-zero-order release of low doses of aspirin; the release should be relatively independent of pH so that drug release is not governed by the pH changes that occur during gastrointestinal transit. (2) The components of the dosage form, including any coatings and films, must not interact or alter with time; the rate of release and total dose released must be unaffected by the conditions or duration of normal storage. (3) Although capsules would be acceptable, the dosage form should preferably be in the form of a tablet to avoid the hazards of tampering that are associated with capsules. (4) The final dosage form must have minimal residence time in the stomach to avoid the gastric irritation associated with aspirin. (5) The process used to prepare the dosage form should avoid non-aqueous solvents, which require extensive and expensive mitigation measures to avoid environmentally unsatisfactory or hazardous conditions, but the aqueous process must not compromise the stability of the water-sensitive aspirin.

Thus, for example, Sothmann and Marttila (US Patent 4,351,825) describe a sustained-release, water-based system using an acrylate/methacrylate copolymer for coating tabletable granules of 50 mg of phenylpropanolamine hydrochloride and 100 mg of verapamil hydrochloride but there is no indication that the medication is released with zero-order kinetics, nor is any duration longer than 3 hours demonstrated. The process is not described in detail, but it does not appear applicable to water-sensitive medicaments such as aspirin. Further, monolithic (also known as matrix) tablets are produced and the problem of gastric residence time is thus not addressed.

Dunn and Lampard (US Patent 4,308,251) describe 650 mg and 850 mg aspirin tablets that exhibit zero-order release in vitro and closely approximate zero-order absorption in vivo, but the tablets are monolithic and the disintegration times shown in the patent are all greater than two hours; the problem of gastric residence time is unrecognized. Further, the medicament tablets are prepared "by dissolving the release controlling agent in suitable organic solvent or solvent mixture such as methylene chloride and denatured alcohol [1:1(v/v)]. Other suitable solvents include, lower aliphatic alcohols such as methanol, isopropanol, n-propanol, etc., acetone and lower aliphatic ketones, such as methylethylketone, chloroform, carbon tetrachloride, ethyl acetate and nonchlorinated hydrocarbons."

Seth (European Application 250,648) describes a multiple unit dosage form of ibuprofen in which microspheres of ibuprofen are coated with Eudragit® E30D ethyl acrylate/methyl methacrylate copolymer, and compressed into tablets containing not less than 600 mg of ibuprofen. The tablets release ibuprofen at an approximately zero-order rate for a period of ten hours; the tablets are said to release a flow of microspheres continously into the intestines from the stomach and this flow is said to be largely independent of the subsequent emptying of the stomach; the coated microspheres are said to display their retard effect throughout the entire duration of transit. The technique for preparing the microspheres requires mixing an aqueous mixture of ibuprofen, microcrystalline cellulose, carboxymethylcellulose and hydroxypropylmethylcellulose, putting the resulting mixture through an extruder and a spheronizer and drying the resulting spheres at 45°C. This is a process that is not feasible with water-sensitive medicaments such as aspirin; in the case of aspirin the amount of salicylic acid formed by hydrolysis during this process would be expected to fall well outside the allowed limits. Seth then describes a process of spray-coating a layer of pure Eudragit®E30D. This process, while manageable when the particles have been deliberately made into hard microspheres, cannot be practiced on irregular granules such as aspirin on a commercial scale. Further Seth does not address the unique problems of low-dose dosage forms; the application states that the need which Seth's discovery satisfies is for a dosage form which contains higher doses than 300 or 400 mg. (page 2, line 17-20). Seth also does not address the question of long-term stability of the release rate.

Schor et al. (US Patent 4,389,393) describe 650 mg aspirin tablets that use hydroxypropylmethylcellulose to provide zero-order release with a duration of 8 hours, and without need for a solvent. However, the lowest release rate described is 65 mg per hour and the tablets are monolithic. It is well known to persons familiar with the art that release rate, tablet size, tablet shape, and dose of medicament have a complex relationship in monolithic tablets; thus a 650 mg matrix tablet cannot be reduced to a 40 to 100 mg dose without unpredictably altering the release rate - perhaps even precluding zero-order release. Additionally, Schor does not recognize the problem of gastric residence time.

Lerk (US Patent 4,244,941) describes a constant-release composition which produces tablets, requires no solvent, and provides a linear release rate approximating zero-order over a period up to five hours. However, the composition only works with highly water-soluble medicaments. Sulfanilamide, which is twice as soluble as aspirin, is the least soluble medicament for which an example is provided, and its release rate is impractically slow. (4.5 mg per hour).

Powell and Patel (US Patent 4,361,545) delineate the importance of zero-order release, and describe a tablet composition that provides zero-order release over periods of 5 to 8 hours for medicaments having the solubility properties of aspirin. The zero-order release depends upon a phenomenon of controlled surface erosion in a monolithic tablet and no tablets containing less than 300 mg of active ingredient are described. Thus, neither the problem of gastric residence time nor the problem of scale down to low-dose is addressed.

Hennig and Kala [Pharmazie 41, 814-815(1986)] describe the coating of aspirin granules of 1.07 mm with an aqueous dispersion of Eudragit®E30D and PEG 6000. A 500-mg aliquot of the resulting particles has a zero-order release rate of 7.16 mg per hour over a period up to 8 hours; however, at 8 hours only 30 to 40% of the aspirin has been released, and there is no indication that the granules so produced could be compressed into a tablet.

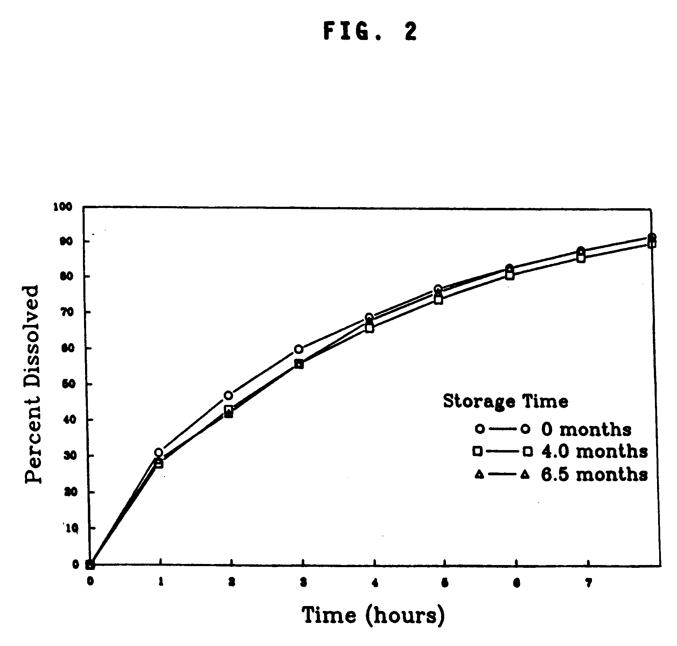

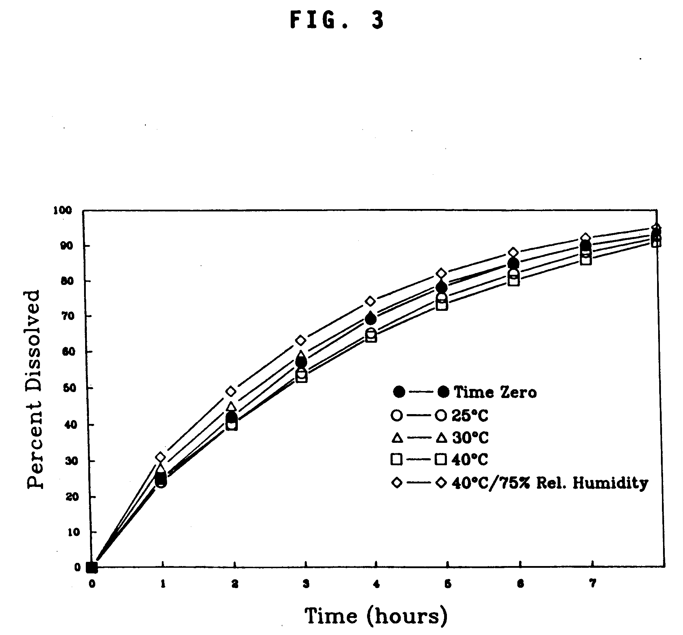

Ventouras (European Application 213083) describes tablets containing 320 and 860 mg of a compound of methylxanthine medicaments in granules coated with Eudragit®E30D, compressed with tableting aids, and coated with Eudragit®E30D, lactose, talc, polysorbate and optionally with pigments. The tablets show zero-order release of methylxanthines over a period of 8 hours. Ventouras indicates that the tablet coating may be modified to control permeability by the inclusion of other water soluble fillers in place of the lactose of the example. Such water-soluble fillers discussed on page 3 paragraph 4 include: "Sodium chloride or a sugar, particularly lactose, fructose or D-mannit, [sic] or sorbitol or polyvinylpyrrolidone or a derivative thereof, or dextrane [sic] compounds of different molecular weight; or a swellable filler, e.g., hydroxypropylmethylcellulose, hydroxyethylcellulose or hydroxypropylcellulose, e.g. Pharmacoat®-603, or an antisticking agent, e.g. talcum, or an emulsifier, e.g. polysorbate (Tween®-80), or a coloring pigment, e.g. indigotin lake or a metal oxide, e.g. iron oxide, such as red iron oxide or yellow oxide, or titanium dioxide; or plasticiser, e.g. polyethylene glycol, such as Lutrol E-400 (BASF)." Since he is not administering aspirin, Ventouras does not address the problems of gastric residence time, and a tablet coated with E30D will remain a monolith. The problem of extending the technology to administer low doses is unappreciated: in fact the release curves for the 2 doses (Figures 2 and 3 of EP Application 213083) indicate a drop in total medicament delivered from about 85% at the 860 mg dose to about 70% at the 320 mg dose.

Kjørnaes and Linnemann (US Patent 4,713,248) describe the coating of potassium chloride crystals in a fluid bed process with Eudragit® E30D, hydroxypropylmethylcellulose and talc. The resulting coated particles may be compressed into tablets to provide tablets that release the same percent of medicament as the coated particles at one hour, indicating that the particles were compressed without substantial fracture of the control-release coating. Kjørnaes and Linnemann also recognize the problem of storage stability, but do not address it with a single-coat particle. They describe a heat treating process which imparts storage stability and provides tablets with approximately zero-order release kinetics up to six hours; however, the particles that are heat treated have a second coating of HPMC and talc applied over the Eudragit® coat. A second patent (US Patent 4,716,041) also to Kjørnaes et al. states (column 6, line 31 to 35 and line 53 to 59) in reference to coatings containing Eudragit®E30D, HPMC, talc and optionally a hydrophobic substance: "In most cases, it has been found that, when subjected to the elevated temperatures necessary to obtain the effect desired above, the inner film layer tends to become tacky (adhesive) causing an undesirable agglomeration of the units...In both instances, ie. both when the substance incorporated in the coating and when the film-forming agent itself causes adhesion, it is therefore, necessary to provide the units with an additional, protective layer which is composed of a substance or a mixture of substances which is anti-adhesive at elevated temperatures and, preferably, also imparts flowability to the coated units." And, in fact, we have observed that if sodium chloride is deleted from the formulation of the present invention, the aspirin granules coated only with Eudragit®, HPMC, and talc tend to agglomerate in the fluid bed coating process even in the absence of additional heat for curing.

Ventouras (US Patent 4,728,513) describes heat-treated granules of a compound of methylxanthine medicaments coated with a 6:1 mixture of ethyl acrylate/methyl methacrylate copolymer (Eudragit®E30D) and ethylcellulose (Aquacoat®ECD-30) followed by a top coat of ethylcellulose. The granules are stable, the release rate being essentially unaffected by storage 1 month at 35°C and only slightly depressed by storage 1 month at 50°C. The granules are compressed into tablets by the use of conventional technology, utilizing art-known fillers, binders, disintegrants, and lubricants. The resulting tablets disintegrate very rapidly and release methylxanthines at approximately zero-order over 8 hours; however, only about 65% of the 900 mg dose is released by 8 hours.

Thus until our invention no one had addressed the problem of efficiently achieving controlled release (5 to 15 mg/hr for 8 hours) of aspirin from a low-dose tablet. Further, the systems described in the prior art that provide essentially zero-order, 5 to 8 hour release for non-aspirin medicaments cannot be extended to the aspirin problem without violating one or more of the requirements satisfied by our invention.

All of the systems described in the prior art that have been applied to aspirin sought stable, non-irritating, or sustained release of doses larger than 300 mg. Sustained-release products generally attempt to generate constant blood levels of a therapeutic agent from one administration of the agent to the next. The focus of our invention is not sustained release, but controlled release. According to our invention, the systemic blood levels of aspirin do not rise above 100 ng/mL at any time during the medication cycle. The duration of release of aspirin is of concern to our invention only indirectly in that it is a dependent variable resulting from the interplay of two required parameters: (1) the total dose must be sufficient to acylate a therapeutically useful proportion of platelet thromboxane synthetase, and (2) the rate of release must be low enough to allow virtually complete presystemic clearance.

One aspect of this invention relates to the use of an aqueous-based formulation for coating aspirin granules which coated granules may be compressed into tablets having approximately zero-order release kinetics for release rates of 5 to 15 mg/hr over a period extending 5 to 8 hours. It is particularly desired that the release kinetics of said granules are substantially unchanged by six month's storage at room temperature, three month's storage at 40°C., or three month's storage at 40°C., and 75% relative humidity.

Another aspect of the invention provides aspirin granules of 0.5 to 1.5 mm particle size coated with 10 to 35%, preferably about 20%, on a dry weight basis, of a formulation containing a methyl methacrylate/ethyl acrylate copolymer, hydroxypropylmethylcellulose (HPMC), sodium chloride, and talc.

### Brief Description of Drawings

Figure 1 shows the release of aspirin in percent of the total dose plotted against the time in hours for the granules of Example 1 and the tablets of Example 2.

Figure 2 shows the release of aspirin in percent release plotted against time in hours for the granules of Example 1 as initially prepared and after storage at room temperature for four months and six and one-half months.

Figure 3 shows the release of aspirin in percent released plotted against time in hours for the granules of Example 1 after storage for three months at 25°, 30°, 40°C, at ambient humidity and 40°C at 75% relative humidity. Ambient humidity is maintained at 35 - 50% relative humidity at 25°C.

Figure 4 shows the excretion of a urinary thromboxane metabolite as a function of time in days versus percent of pretreatment levels for subjects receiving placebo and subjects receiving tablets of the invention.

Figure 5 shows the serum levels of thromboxane in subjects receiving placebo and receiving tablets of the invention as a function of time in days versus percent of pre-treatment levels.

Figure 6 shows the urinary excretion of a prostacyclin metabolite at 4 days and at 21 days in control and medicated subjects as a percentage of pre-treatment levels.

One aspect of the invention resides in an aqueous based formulation for coating aspirin granules to provide coated granules that may be compressed into tablets and that show approximately zero-order release kinetics for release rates of 5 to 15 mg/hr over a period extending 5 to 8 hours. The formulation consists essentially of (a) from about 40 to about 60 parts of a 70:30 copolymer of ethyl acrylate and methyl methacrylate of average molecular weight 800,000; (b) from about 10 to about 20 parts of USP 2910 hydroxypropylmethylcellulose of ASTM viscosity 3 to 15 mPa (3 to 15 cps)(c) from about 1 to about 12 parts of sodium chloride; (d) from about 20 to about 45 parts of talc USP and (e) from about 200 to about 900 parts of water.

A preferred embodiment of the coating formulation consists of about 48 parts of acrylate copolymer, about 16 parts of hydroxypropylmethylcellulose, about 3.2 parts of sodium chloride, abut 32 parts of talc and about 396 parts of water. The acrylate/methacrylate copolymer is commercially available as a 30% aqueous dispersion from Rohm Pharma GmbH, Darmstadt (Federal Republic of Germany) under the name Eudragit®NE30D (formerly known as E30D). The preferred hydroxypropylmethylcellulose carries the USP designation 2910 6 mPa (6 cps) which indicates 28- 30% by weight methoxyl groups, 7 - 12% by weight hydroxypropyl groups, and an average molecular weight such that a 2% aqueous solution has an ASTM viscosity of 6 mPa (6 cps) at 20°C. A hydroxypropylmethylcellulose which meets this criterion is available from the Dow Chemical Company (USA) as Methocel®E and Shin-etsu Limited (Japan) as Pharmacoat®606. The talc of the preferred embodiment has a median particle size of 3µm and is available from Cypruss Industrial Minerals Company(USA) as Altalc®500.

It is contemplated that coating compositions comprising HPMC and ethylcellulose may be utilized to produce coated aspirin granules having a release rate of 5 to 15 mg/hr, but we have observed that the resulting coated granules cannot be compressed into tablets that release aspirin at 5 to 15 mg/hr without an unacceptable, large, initial burst of aspirin. The HPMC/ethylcellulose coated granules could, however, be put into capsules by methods conventional in the art. A capsule containing 40 to 100 mg of the coated aspirin, although less desirable because of it greater susceptibility to tampering, would function to deliver aspirin at the combination of the correct rate and dose to provide the optimum differentiation between inhibition of TX and inhibition of PC.

In a further composition aspect, the invention resides in a controlled-release aspirin granule of particle size 0.5 to 1.5mm coated with 10 to 35%, preferably about 20°, on a dry weight basis, of a formulation containing from about 40 to about 60, preferably about 48, parts of a 70:30 copolymer of ethyl acrylate and methyl methacrylate of average molecular weight 800,000 from about 10 to about 20, preferably about 16, parts of USP 2910 HPMC, preferably of 3-15 mPa (3-15 cps) viscosity, most preferably of 6 mPa (6 cps) viscosity, from about 1 to about 12, preferably about 3.2 parts of sodium chloride and from about 20 to about 45, preferably about 32, parts of talc USP, preferably of particle size 3µm.

The function of the acrylate/methacrylate copolymer or other coating polymer of the coating formulation is to provide a permeable, but insoluble, shell that is unaffected by pH, that does not chemically interact with aspirin, and that will limit the rate of dissolution of aspirin. Its low glass transition temperature provides deformability when used to produce a tablet. The coating formulation can be applied in an aqueous-based spray coating operation. Other polymers having those properties would provide acceptable equivalents. The HPMC could, in principle, be replaced by any water-soluble, hydrophilic polymer to modulate the release of aspirin through the acrylate/methacrylate coat. Sodium chloride appears to function to both as a permeability enhancer for the polymeric coat and as an aid to processing in the fluid bed technique used to coat granules; as such it prevents agglomeration of the fluid bed. Other water-soluble, pharmacologically innocuous salts would function in place of sodium chloride provided that they form crystalline inclusions in the polymer coat. Although talc is preferred to reduce tackiness and provide bulk, many pharmacologically innocuous. water-insoluble, anti-adhesive coating aids and pigments are known in the art: colloidal silicon dioxide, iron oxide, titanium dioxide etc.

In a further composition aspect, the invention resides in a rapidly disintegrating, low-dose, controlled-release aspirin tablet containing from 40 to 100 mg of aspirin coated as above and such other excipients, binders, glidants, lubricants, diluents, plasticizers, film coatings, tableting aids, and disintegration enhancers as may be needed to maintain the release rate of about 5 to about 15 mg per hour and to provide a disintegration time of less than 15 minutes. To produce such a tablet, we have found it useful to utilize filler granules for the tableting process. For this purpose, any pharmaceutically inert granules that are roughly comparable in size to the coated aspirin granules and that will deform or crush in preference to the coated aspirin granules under the compression forces of tableting may be used. It is advantageous to have a sufficient size distribution of the filler granules so as to fill in the interstices and provide a mechanically stable tablet.

To provide filler granules having those characteristics, we have found the following formulation to be particularly advantageous: 340 parts of hydrous lactose USP, 88 parts of microcrystalline cellulose USP, 25 parts of an 11 % mixture of sodium carboxymethylcellulose and microcrystalline cellulose, and 39 parts of pregelatinized starch USP/NF bound with 18 parts of USP 2910 HPMC 15 mPa (15 cps). The microcrystalline cellulose USP of preferred 50 µm average particle size is commercially available from FMC as Avicel® PH101. The microcrystalline cellulose containing 11± 2.7% carboxymethylcellulose is available from FMC as Avicel® RC-581. The pregelatinized starch is available from Colorcon Inc. (USA). The hydroxypropylmethylcellulose 15 cps is available from Dow Chemical Company (USA) as Methocel® E and from Shin-etsu (Japan), as Pharmacoat®615. Alternatively, one may utilize a commercially available filler granule such as Ludipress® (BASF), a lactose/PVP/crospovidone granule of particle size about 100 to 400 µm.

A preferred embodiment of the tablet consists of 219 mg of the filler granules described above, 92.4 mg of the coated aspirin granules described above, 13.4 mg of sodium starch glycolate, 3.35 mg of talc USP/NF, and 6.7 mg of stearic acid USP/NF. The sodium starch glycolate is commercially available from Generichem (USA) as Primogel® or from Mendel (USA) as ExploTab®.

Although not necessary to the practice of the invention, the tablets may have a film coating to minimize mechanical breakdown and provide a more readily swallowed dosage form for the patient. Any film coating that would minimize mechanical breakdown and provide a readily swallowed dosage form without interfering with the release kinetics of the particles or the disintegration rate of the tablet in the stomach would be suitable. However, we have found a coating consisting of about 62 parts of USP 2910 hydroxypropylmethylcellulose of 6 mPa (6 cps) viscosity, about 12 parts of polyethylene glycol (PEG) 8000 USP/NF, about 21 parts of titanium dioxide USP, and about 4 parts of talc, USP, to be particularly advantageous when applied at a rate of about 10 mg per tablet. When this film coating is applied, a preferred tablet of the invention is obtained; it contains about 75 mg of aspirin, about 8.5 mg of acrylate copolymer, about 0.56 mg of sodium chloride, about 9.2 mg of hydroxypropylmethylcellulose, about 5.6 mg of 3 µm (500 mesh) talc, about 146 mg of lactose, about 38 mg of microcrystalline cellulose, about 11 mg of 11% sodium carboxymethylcellulose in microcrystalline cellulose, about 10 mg of pregelatinized starch, and about 13 mg of sodium starch glycolate, and is coated with about 6.25 mg of HPMC, abut 1.22 of PEG 8000, about 2.13 mg of titanium dioxide, and about 0.4 mg of talc.

In a process aspect the invention resides in a process for preparing near-zero-order, controlled-release aspirin granules which comprises the aqueous spray-coating of aspirin granules, preferably of particle size 0.5 to 1.5mm, with a suspension of about 40 to about 60 preferably about 48, parts of a 70:30 copolymer of ethyl acrylate and methyl methacrylate of molecular weight 800,000; and about 10 to about 20, preferably about 16, parts of USP 2910 HPMC, preferably of 6 mPa (6 cps) HPMC; about 1 to about 12, preferably about 3.2 parts of sodium chloride; and from about 20 to about 45, preferably about 32 parts, of talc, preferably having a median particle size of about 3µm; in about 200 to about 900, preferably about 396, parts of water. The process provides a controlled-release coating that, after drying, constitutes from about 10 to about 35%, preferably about 20%, of the weight of the granule.

In a further process aspect the invention relates to a process for preparing a rapidly-disintegrating, low-dose, controlled-release aspirin tablet. The process comprises the steps of (1) preparing coated aspirin granules by dissolving about 10 to about 20 USP 2910 hydroxypropylmethylcellulose of 3 to 15 mPa (3 to 15 cps) viscosity in about 90 to about 180 parts of water, suspending about 20 to about 45 parts of talc USP having a median particle size of 3µm in a solution of about 1 to about 12 parts of sodium chloride in about 40 to about 580 parts of water, combining both with about 130 to about 200 parts of a 30% aqueous emulsion of a 70:30 copolymer of ethyl acrylate and methyl methacrylate and applying the coating mixture to about 580 parts of 0.84074-0.59436 mm, (20-30 mesh) aspirin granules by a suitable air-suspension coating method to provide discrete, coated granules; (2) providing filler granules that are pharmaceutically inert and roughly comparable in size to the aspirin granules of part (1); (3) compressing a homogenous mixture of about 65 to about 90 parts of said filler granules, about 27 parts of said coated aspirin granules, and such other glidants, disintegrants and processing aids as may be required to produce tablets that disintegrate in less than 15 minutes, that deliver aspirin at a rate of 5 to 15 mg/hr and that contain from about 40 to about 100 mg of aspirin each; and (4) optionally film coating said tablets with a rapidly water-soluble film.

The filler granules may be purchased (eg. Ludipress®) or prepared by blending about 340 parts of hydrous lactose USP, about 88 parts of microcrystalline cellulose USP of 50 µm average particle size, about 25 parts of an 11% mixture of sodium carboxymethylcellulose in microcrystalline cellulose, and about 39 parts of pregelatinized starch USP/NF in a fluid bed granulator and applying a binder of about 18 parts of USP 2910 hyroxypropylmethylcellulose of 15 mPa (15 cps) viscosity in about 239 parts of water at 35 - 50°.

The preferred disintegrant for the production of compressed tablets is about 4 parts of sodium starch glycolate; the preferred glidant is about 2 parts of stearic acid NF; and the preferred processing aid is about 1 part of talc USP.

The preferred film coating process comprises optionally film coating said tablets with a homogenous mixture of about 62 parts of USP 2910 hydroxypropylmethylcellulose of 6 mPa (6 cps) viscosity, about 12 parts of polyethylene glycol 8000 USP/NF, about 21 parts of titanium dioxide USP, and about 4 parts of talc USP in about 900 parts of water at about 50 - 65°C such that the dry weight of film coat on each tablet is about 10 mg.

The following examples will further illustrate the invention without, however, limiting it thereto.

### Example 1

A 10% solution of hydroxypropylmethylcellulose(HPMC) was prepared by heating 4.952L of water to 70°C and adding 1.238 kg of HPMC 2910 6 mPa (6 cps) with agitation. A second 4.952L of cold water was added, and stirring was continued until a lump-free solution was obtained. The solution was allowed to deaerate and cool to room temperature. To 12.10 L of water was added 0.2475 kg sodium chloride. When the sodium chloride had dissolved 2.475 kg of talc USP 3µm was added with stirring to form a uniform dispersion. 12.38 kg of Eudragit®NE30D was passed through a 0.4191 mm, (40 mesh) screen into a suitable container and the deaerated, cooled 10% HPMC solution was added with moderate agitation, followed by the talc/sodium chloride dispersion. The mixture was stirred for at least 30 minutes prior to coating. Mild agitation was maintained during the coating process.

A Glatt CPCG-60 fluidized bed coater, equipped with an 18" Wurster column, was preheated to about 45°C. The column was charged with 33 kg of 0.84074-0.59436 mm, (20-30 mesh) aspirin granules and fluidized to an appropriate level (500 - 600 cu. ft./minute). 38.35 kg of the coating suspension prepared as above was applied through a 1.2mm nozzle (atomized by 2.0 bar air pressure) at a rate of 160-200 gram per minute to maintain a product temperature of about 25°C. When the coating suspension was completely consumed, the inlet air temperature was increased to 55°C and the material was dried and cured for 60 minutes. The coated granules were discharged from the fluid bed, passed through a 1.1913 mm, (16 mesh) screen as a check for agglomerates, and stored until needed for tableting.

### EXAMPLE 2

### Preparation Of The Tablets

### Fluid bed Preparation of filler granules

A 7% hydroxypropylmethylcellulose binder solution was prepared by heating 30 kg of water to 70°C and adding 3.00 kg of HPMC 2910 15 mPa (15cps) with rapid agitation. Ten kg of cold water was added and the mixing was continued until a lump-free solution was obtained. The solution was allowed to deaerate and cool to below 35°C. 55.2 kg of lactose, 14.4 kg of microcrystalline cellulose, 4.0 kg of Avicel® RC 581, (an 11% mixture of sodium carboxymethyl cellulose in microcrystalline cellulose) and 6.4 kg pregelatinized starch 1500 were separately passed through a 0.84074 mm, (20 mesh) stainless steel screen to remove any large particles. A fluid bed granulator (Glatt GPCG-60) was heated to 40°C and the lactose, microcrystalline cellulose, Avicel® RC581, and starch 1500 were transferred into the preheated granulator. The material was fluidized for 2 minutes and the HPMC binder solution was applied at 650 - 850 g per minute through three 1.8 mm nozzles with an inlet air temperature of 35 - 50°C. When the application was complete, the product was dried at 80°C until a "loss on drying" test showed less than 3% moisture. After drying,, the filler granules were passed through a 1.4097 mm, (14 mesh screen) and stored until needed for tableting.

### Tableting

5.44 kg of sodium starch glycolate [Explotab® low pH] was passed through a 0.59436 mm, (30 mesh screen) to remove agglomerates and then combined with 37.5 kg of coated aspirin granules from Example 1 and 88.9 kg of filler granules. The mixture was blended in a twin shell blender for 10 minutes and 1.36 kg of USP-talc that had been put through a 0.24892 mm, (60 mesh) screen was added and blended 4 or 5 minutes. 2.72 kg of stearic acid USP was passed through a 0.24892 mm, (60 mesh) screen, added to the mixture and blended for a further 5 mimutes. The mixture was compressed into tablets on a Manesty Betapress® with 3/8" standard concave tooling to produce tablets weighing 335± 10 mg, having a hardness of 3 to 10 KP and a thickness of 0.445 cm, ± 0.013 cm, (0.175 inches ±0.005 inches).

### Film Coating

12.8 L of water was heated to 70° and 522 g of polyethylene glycol 8000 USP (Carbowax® 800) was added with stirring, followed by 2.68 kg of hydroxypropylmethylcellulose 2910 6 mPa (6 cps) (Pharmacoat® 606). Agitation was continued and 25.7 liters of cold water was added. A portion of the solution was combined with 912 g of titanium dioxide USP and passed through an Eppenbach homogenizing mill at 0.013 cm, (0.005 inches) to disperse and homogenize the mixture. The homogenized mixture was reunited with the remaining solution and mixed. 132 kg of uncoated tablets was placed in a 121.9 cm, (48 inch) coating pan (Accela Cota®) and preheated to 40 - 50°C. The film coating was sprayed through two guns, nozzle size 1.092 mm, (0.043 inch), needle 0.838 mm, (0.033 inch), at 175 to 225 g per minute continously through each gun, onto the tablets which were rotated at 9 to 12 rpm with inlet air at 50 to 65°C at 2,000 cubic feet per minute. A film of approximately 10 mg per tablet was applied to yield tablets having a weight of 345 ± 10 mg.

### EXAMPLE 3

### Analytical Determination Of Release Rate From Granules

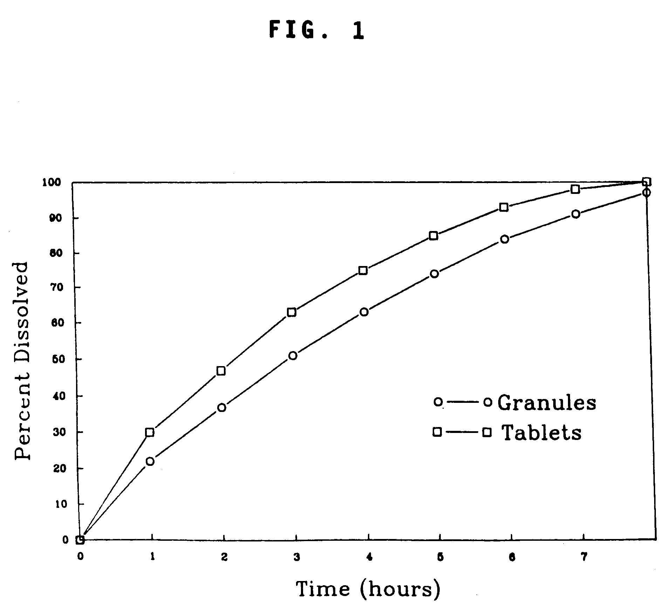

The general method and apparatus used are described in USP XXI(711) apparatus 2, rotating paddle. A buffer of pH 6.0 was prepared from 1600 mL of water, 13.5 mL of 85% phosphoric acid and 15.12 g of sodium chloride adjusted to pH 6.0 ± 0.05 with 5N sodium hydroxide. A standard was prepared by dissolving 50 mg of aspirin in 1 mL of ethanol and diluting to 100 mL with pH 6.0 buffer. To prepare a standard dilution of about 0.15 mg aspirin per mL, 15.0 mL of the foregoing solution was diluted to 50 mL with pH 6.0 buffer. The assay was carried out in 500 mL of pH 6.0 buffer at 37±0.5° with a paddle stirring speed of 100 rpm. A granule sample equivalent to 75 mg of aspirin was introduced into the apparatus and 10.0 mL aliquots were removed at 1 hour intervals over a period of 8 hours. The quantity of aspirin in the aliquot was determined by spectrophotometric comparison with the standard dilution in 1 cm cells at 266 nm. Figure 1 shows a plot of % released versus time in hours for the granules of Example 1. The release profile was not significantly different at pH 1, 4.5, or 7.4 Figures 2 and 3 show a plot of % released versus time for the granules of Example 1 after storage under various conditions for different times.

### EXAMPLE 4

### Analytical Determination Of Release Rate From Tablets

The method described in Example 3, was used but the granule sample was replaced by a tablet containing 75 mg of aspirin. The resulting curve derived from tablets according to Example 2 is shown in Figure 1.

### EXAMPLE 5

### Analytical Determination of The Disintegration Rate Of Tablet

The disintegration rate of a tablet prepared according to Example 2 was analyzed using the procedure of USP XXI (701) using the disk method and water as the emersion fluid. The disintegration time of tablets prepared according to Example 2 is less than 5 minutes.

### EXAMPLE 6

### Determination of Inhibition of TX and PC in Humans

Forty-eight male volunteers were randomly assigned to one of four groups: (1) group A received 75 mg of aspirin orally as a solution each morning for 21 days; (2) group B received orally one tablet of the invention containing 75mg of aspirin each morning for 21 days; (3) group C received orally one tablet of the invention containing 50 mg of aspirin each morning for 21 days; (4) group D received orally one placebo tablet each morning for 21 days. The tablets were taken with 113.7 ml, (four ounces) of water; the solution was given in 56.83 ml, (two ounces) of water followed by a second two ounces of water.

The urine from each individual was collected over a 24 hour period on days 1, 8, 15, and 22. Urine samples to be pooled were stored at 0 - 10°C during the course of the 24 hour collection, then each individual's 24 hour sample collection was pooled and stored at -20°C until analysis.

Approximately 2 mL of blood was drawn by venipuncture from the forearm of each indiviual just before the administration of aspirin on days 2, 9, 16 and 23. The blood was allowed to clot at 37°C for 45 to 60 minutes and the serum was withdrawn following centrifugation. It was stored at -20°C until analysis.

In a second study, 52 male volunteers were randomly assigned to one of three groups: (1) group E (n=16) received a 50-mg bolus dose of aspirin as a solution directly into the duodenum by gastric tube each morning for four days; (2) group F (n=20) received a 50-mg dose of aspirin solution intraduodenally as an infusion over the course of five hours each morning for four days; (3) group G (n=16) received a 50-mg dose of aspirin solution intraduodenally as an infusion over the course of ten hours each morning for four days. Urine was collected on day 5 and day 10 as before, pooled, and stored at -20° C until analysis.

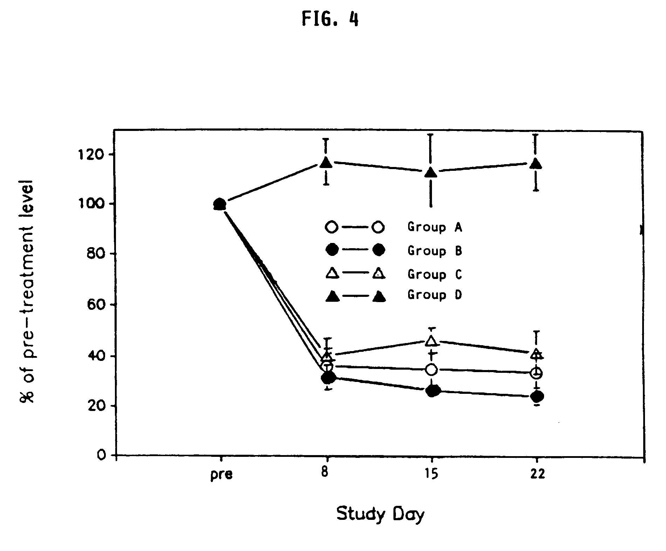

Analysis of urinary 2,3-dinorthromboxane B₂ was carried out by the procedure of Lawson et al. [Analytical Biochemistry 150, 463-470 (1985)] with minor modifications. Analysis of urinary 2,3-dinor-6-ketoprostaglandin F_{1α} was carried out by the procedure of FitzGerald et al. [Advances in Prostaglandin, Thromboxane and Leukotriene Research 15, 87-90 (1985)] with minor modifications. Analysis of serum thromboxane B₂ was carried out by the method of Lawson et al. [Analytical Biochemistry 155, 198-205 (1985)]. Figure 4 shows the amount of 2,3-dinor-thromboxane B₂ in the urine of subjects in each of groups A-D as a percentage of pretreatment levels at each of days 8, 15 and 22. 2,3-Dinorthromboxane B₂ is a metabolite of the thromboxane A₂. It arises primarily from platelet cyclooxygenase, although up to 20% of urinary 2,3-dinor-thromboxane B₂ arises from non-platelet sources.

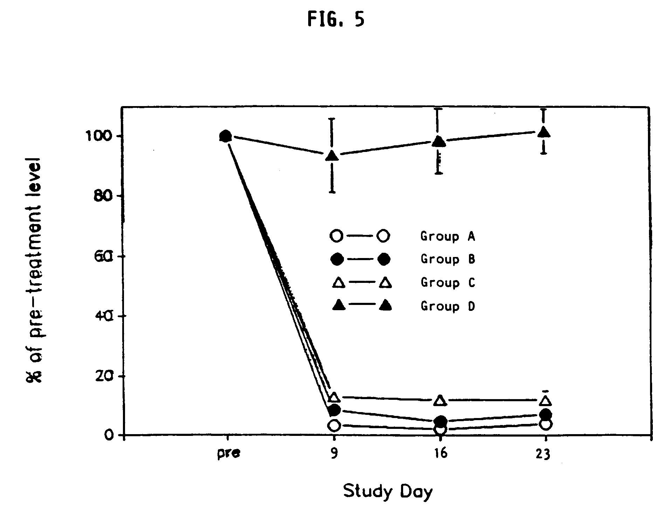

Figure 5 shows the amount of thromboxane B₂ in the serum of subjects as a percentage of pretreatment levels at each of days 2, 9, 16 and 23. Thromboxane B₂ is a metabolite of Thromboxane A₂ that, in the serum, arises primarily from platelet cyclooxygenase.

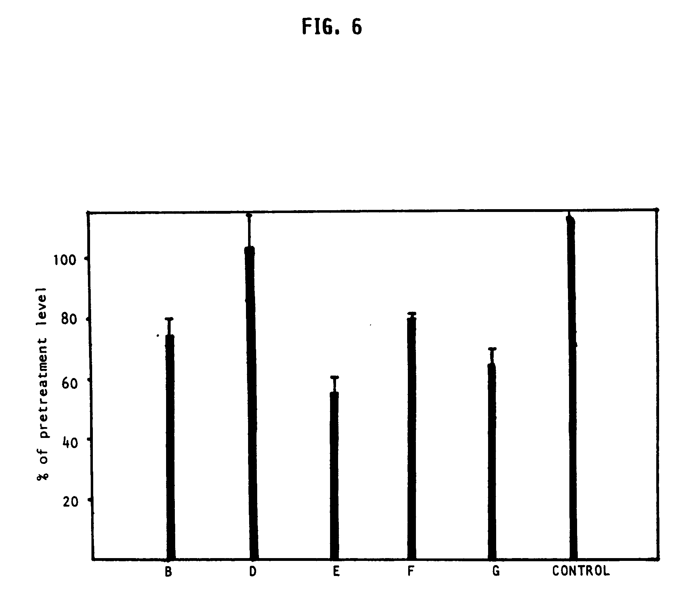

Figure 6 shows the amount of 2,3-dinor-6-ketoprostaglandin F_{1α} in the urine of subjects as a percentage of pretreatment levels at day 22 of the first study and at day 5 of the second study. 2,3-Dinor-6-ketoprostaglandin F_{1α} is a metabolite of prostacyclin (prostaglandin-I₂).

A comparison of figures 4,5 and 6 shows that 75 mg of controlled-release aspirin of the invention as well as 75 mg of aspirin solution lower urinary thromboxane to about 25% of pretreatment levels, which is virtually complete suppression of platelet cyclooxygenase. Fifty milligrams of controlled-release aspirin lowers urinary TX to about 40% of pretreatment levels. A similar result is seen in the case of serum TX levels although the suppression is more evident due to lower residual, non-platelet TX in serum. In contrast, urinary prostaglandin levels are only reduced by about 25% by the 75 mg controlled-release formulation whereas soluble aspirin causes a reduction of about 50%. Thus the dose of about 40 mg to about 100 mg, preferably about 75 mg, of aspirin delivered at a rate of about 5 mg/hr to about 15 mg/hr, preferably about 8 mg/hr to about 10 mg/hr, provides the optimal separation of inhibition of platelet cyclooxygenase from inhibition of endothelial cyclooxygenase.

## Claims (Claims for the following Contracting State(s): AT, BE, CH, DE, DK, FR, GB, IT, LI, NL, SE)

1. The use of an aqueous-based formulation for coating aspirin granules to provide coated granules that show approximately zero-order release kinetics for release rates of 5 to 15 mg/hr over a period extending five to eight hours, said formulation consisting essentially of (a) from 40 to 60 parts of a 70:30 copolymer of ethyl acrylate and methyl methacrylate of average molecular weight 800,000; (b) from 10 to 20 parts of hydroxypropylmethylcellulose of ASTM viscosity 3-15 mPa (3 to 15 cps) and having a hydroxypropyl content of 7 to 12 weight percent and a methoxyl content of 28 to 30 weight percent; (c) from 1 to 12 parts of sodium chloride; (d) from 20 to 45 parts of talc USP; and (e) from 200 to 900 parts of water.

2. Aspirin granules of particle size 0.5 to 1.5 mm coated with 10 to 35% on a dry weight basis of a formulation according to claim 1.

3. A rapidly disintegrating, low-dose, controlled-release aspirin tablet comprising from 40 to 100 mg of aspirin granules coated with a formulation according to claim 1.

4. An aspirin tablet according to claim 3, which includes one or more of exipients, binders, glidants, lubricants, diluents, plasticizers, film coatings, tableting aids, disintegration enhancers, and filler granules so that the tablet disintegrates within 15 minutes and provides a release rate of aspirin of 5 to 15 mg per hour.

5. A process for preparing near-zero-order, controlled-release aspirin granules which comprises the aqueous spray-coating of aspirin granules with a suspension of 40 to 60 parts of a 70:30 copolymer of ethyl acrylate and methyl methacrylate of molecular weight 800,000, 10 to 20 parts of USP 2910 hydroxypropylmethylcellulose, 1 to 12 parts of sodium chloride and 20 to 45 parts of talc in 200 to 900 parts of water.

6. A process according to claim 5, wherein the total weight of dry solids spray-coated onto said aspirin granules is from 10 to 35% of the weight of said granules.

7. A process for preparing a low-dose, rapidly disintegrating, controlled-release aspirin tablet which comprises the steps of:
(1) preparing coated aspirin granules by dissolving 10 to 20 parts of USP 2910 hydroxypropylmethylcellulose of 3 to 15 mPa (3 to 15 cps viscosity) in 90 to 180 parts of water, suspending 20 to 45 parts of talc USP having a median particle size of 3 µm in a solution of 1 to 12 parts of sodium chloride in 40 to 580 parts of water, combining both with 130 to 200 parts of a 30% aqueous emulsion of a 70:30 copolymer of ethyl acrylate and methyl methacrylate and applying the coating mixture to 580 parts of 0.84074-0.59436 mm, mesh opening (20-30 mesh) aspirin granules by suitable airsuspension coating method to provide discrete, coated granules;
(2) providing filler granules that are pharmaceutically inert and roughly comparable in size to the aspirin granules of part(1); and
(3) compressing a homogenous mixture of 65 to 90 parts of said filler granules, 27 parts of said coated aspirin granules, and such other glidants, disintegrants and processing aids as may be required to produce tablets that disintegrate in less than 15 minutes, that deliver aspirin at a rate of 5 to 15 mg/hr and that contain from 40 to 100 mg of aspirin each.

8. A process according to claim 7, which includes
(4) film coating said tablets with a rapidly water soluble film.

9. Coated aspirin granules, in which the aspirin granules have been coated with a formulation comprising a coating polymer providing a permeable, but insoluble shell which is unaffected by pH, does not chemically interact with the aspirin granules, and limits the rate of dissolution of the aspirin granules; a water-soluble hydrophilic polymer adapted to modulate the release of aspirin by virtue of the permeable, but insoluble shell; a water-soluble pharmacologically innocuous salt to provide crystalline inclusions in the shell; and an innocuous, water-insoluble, anti-adhesive coating aid to reduce tackiness in forming the insoluble shell, and water in an amount to provide an aqueous-based formulation for coating said shell on said aspirin granules, wherein the components of said formulation are chosen and used in such amounts to enable the aspirin granules to be released at a rate of from 5 to 15 mg/hr over a period extending from five to eight hours.

10. Coated aspirin granules according to claim 9 formed into a tablet, wherein the coating polymer which provides the permeable but insoluble shell has a low glass transition temperature so as to provide deformability to the shell.

11. The use of aspirin granules or aspirin tablets according to any one of claims 2-4, 9 and 10 or prepared by a process according to any one of claims 5-8 for making a medicament for treating or preventing vascular occlusive disease in humans.

## Claims (Claims for the following Contracting State(s): ES, GR)

1. A process for preparing near-zero-order, controlled-release aspirin granules which comprises aqueous spray-coating of aspirin granules with a suspension of 40 to 60 parts of a 70:30 copolymer of ethyl acrylate and methyl methacrylate of molecular weight 800,000, 10 to 20 parts of USP 2910 hydroxypropylmethylcellulose, 1 to 12 parts of sodium chloride and 20 to 45 parts of talc in 200 to 900 parts of water.

2. A process according to claim 1, wherein the total weight of dry solids spray-coated onto said aspirin granules is from 10 to 35% of the weight of said granules.

3. A process according to claim 2, wherein the total weight of dry solids is 20% of the weight of said aspirin granules.

4. A process for preparing a low-dose, rapidly disintegrating, controlled-release aspirin tablet which comprises:
(1) preparing coated aspirin granules by dissolving 10 to 20 parts of USP 2910 hydroxypropylmethylcellulose of 3 to 15 mPa (3 to 15 cps viscosity) in 90 to 180 parts of water, suspending 20 to 45 parts of talc USP having a median particle size of 3 µm in a solution of 1 to 12 parts of sodium chloride in 40 to 580 parts of water, combining both with 130 to 200 parts of a 30% aqueous emulsion of a 70:30 copolymer of ethyl acrylate and methyl methacrylate and applying the coating mixture to 580 parts of 0.84074-0.59436 mm, mesh opening (20-30 mesh) aspirin granules by suitable airsuspension coating method to provide discrete, coated granules;
(2) providing filler granules that are pharmaceutically inert and roughly comparable in size to the aspirin granules of part(1); and
(3) compressing a homogenous mixture of 65 to 90 parts of said filler granules, 27 parts of said coated aspirin granules, and such other glidants, disintegrants and processing aids as may be required to produce tablets that disintegrate in less than 15 minutes, that deliver aspirin at a rate of 5 to 15 mg/hr and that contain from 40 to 100 mg of aspirin each.

5. A process according to claim 4, which comprises the steps of:
(1) preparing coated aspirin granules by dissolving 22 parts of USP 2910 hydroxypropylmethylcellulose of 6 mPa (6 cps viscosity) in 174 parts of water, suspending 44 parts of talc USP having a median particle size of 3 µm in a solution of 4 parts of sodium chloride in 213 parts of water, combining both with 218 parts of a 30% aqueous emulsion of a 70:30 copolymer of ethyl acrylate and methyl methacrylate and applying the coating mixture to 580 parts of 0.84074-0.59436 mm, (20-30 mesh) aspirin granules;
(2) preparing filler granules by blending 340 parts of hydrous lactose USP, 88 parts of microcrystalline cellulose USP of 50 µm average particle size, 25 parts of an 11% mixture of sodium carboxymethylcellulose in microcrystalline cellulose, and 39 parts of pregelatinized starch USP/NF in a fluid bed granulator and applying a binder of 18 parts of USP 2910 hydroxypropylmethylcellulose of 15 mPa (15 cps) viscosity in 239 parts of water at 35 to 50°C; and
(3) compressing a homogenous mixture of 65 parts of said filler granules, 27 parts of said coated aspirin granules, 4 parts of sodium starch glycolate USP, 1 part of talc USP and 2 parts of stearic acid NF in a standard tablet press to produce tablets weighing about 335 mg and containing 75 mg of aspirin each.

6. A process according to claim 4 or 5, which includes
(4) film coating said tablets with a rapidly water soluble film.

7. A process according to claim 6, wherein
(4) comprises film coating said tablets with a homogenous mixture of 62 parts of USP 2910 hydroxypropylmethylcellulose of 6 mPa (6 cps viscosity), 12 parts of polyethylene glycol 8000 USP/NF, 21 parts of titanium dioxide USP, and 4 parts of talc USP in 900 parts of water at 50-65°C. such that the final weight of each tablet is 345 mg.

8. A process for preparing approximately zero-order, controlled-release aspirin granules which comprises spray-coating aspirin granules with a formulation comprising a coating polymer providing a permeable, but insoluble shell which is unaffected by pH, does not chemically interact with the aspirin granules, and limits the rate of dissolution of the aspirin granules; a water-soluble hydrophilic polymer adapted to modulate the release of aspirin by virtue of the permeable, but insoluble shell; a water-soluble pharmacologically innocuous salt to provide crystalline inclusions in the shell; and an innocuous, water-insoluble, anti-adhesive coating aid to reduce tackiness in forming the insoluble shell, and water in an amount to provide an aqueous-based formulation for coating said shell on said aspirin granules, wherein the components of said formulation are chosen and used in such amounts to enable the aspirin granules to be released at a rate of from 5 to 15 mg/hr over a period extending from five to eight hours.

9. A process according to claim 8, which includes admixing the coated aspirin granules with filler granules which are pharmaceutically inert and roughly compatible in size to the coated aspirin granules and compressing a homogenous mixture of the filler granules with the coated aspirin granules and such other glidants, disintegrants and processing aids as may be required to produce tablets which disintegrate in less than 15 minutes.

## Patentansprüche (Patentansprüche für folgende(n) Vertragsstaat(en): AT, BE, CH, DE, DK, FR, GB, IT, LI, NL, SE)

1. Verwendung einer Formel auf Wasserbasis zum Überzug von Aspirinkörnchen, so daß überzogene Körnchen vorgesehen werden, die bei Freisetzungsgeschwindigkeiten von 5 bis 15 mg/Stunde über einen Zeitraum von fünf bis acht Stunden eine Freisetzungskinetik von ungefähr Null aufweisen, wobei die Formel im wesentlichen folgendes umfaßt: (a) 40 bis 60 Anteile eines 70:30 Copolymers aus Ethylacrylat und Methylmethacrylat mit einem durchschnittlichen Molekulargewicht von 800.000; (b) 10 bis 20 Anteile Hydroxypropylmethylcellulose mit einer ASTM-Viskosität von 3-15 mPa (3-15 cps) und mit einem Hydroxypropylgehalt von 7 bis 12 Gewichtsprozent und mit einem Methoxylgehalt von 28 bis 30 Gewichtsprozent; (c) 1 bis 12 Anteile Natriumchlorid; (d) 20 bis 45 Anteile Talk USP; und (e) 200 bis 900 Anteile Wasser.

2. Aspirinkörnchen mit einer Partikelgröße von 0,5 bis 1,5 mm, die auf Trockengewichtsbasis mit 10 bis 35% einer Formel gemäß Anspruch 1 überzogen sind.

3. Schnell auflösende, niedrigdosierte Aspirintablette mit gesteuerter Freisetzung, die 40 bis 100 mg Aspirinkörnchen umfaßt, die mit einer Formel gemäß Anspruch 1 überzogen sind.

4. Aspirintablette nach Anspruch 3, die einen oder mehrere der folgenden Bestandteile aufweist: Bindemittel, Gleitmittel, Schmiermittel, Verdünnungsmittel, Plastifizierungsmittel, Filmüberzüge, Tablettierhilfsstoffe, Zerfallsbeschleunigungsmittel und Füllkornchen, so daß sich die Tablette innerhalb von 15 Minuten auflöst und eine Freisetzungsgeschwindigkeit des Aspirins von 5 bis 15 mg pro Stunde aufweist.

5. Verfahren zur Herstellung von Aspirinkörnchen mit einer Ordnung von nahezu Null und mit gesteuerter Freisetzung, wobei das Verfahren den Wasserspritzüberzug der Aspirinkörnchen mit einer Suspension vorsieht, die folgendes umfaßt: 40 bis 60 Anteile eines 70:30 Copolymers aus Ethylacrylat und Methylmethacrylat mit einem Molekulargewicht von 800.000, 10 bis 20 Anteile USP 2910 Hydroxypropylmethylcellulose, 1 bis 12 Anteile Natriumchlorid und 20 bis 45 Anteile Talk in 200 bis 900 Anteilen Wasser.

6. Verfahren nach Anspruch 5, wobei das Gesamtgewicht der auf die Aspirinkörnchen gespritzten Trockenstoffe 10 bis 35 Gewichtsprozent der Körnchen darstellt.

7. Verfahren zur Herstellung einer niedrigdosierten, sich schnell auflosenden Aspirintablette mit gesteuerter Freisetzung, wobei das Verfahren die folgenden Schritte umfaßt:
(1) Vorbereitung überzogener Aspirinkörnchen durch Auflösen von 10 bis 20 Anteilen USP 2910 Hydroxypropylmethylcellulose mit einer Viskosität von 3 bis 15 mPa (3 bis 15 cps) in 180 Anteilen Wasser, wobei 20 bis 45 Anteile Talk USP mit einer mittleren Partikelgröße von 3 µm in einer Lösung von 1 bis 12 Anteilen Natriumchlorid in 40 bis 580 Anteilen Wasser aufgelöst werden, wobei beide mit 130 bis 200 Anteilen einer 30-prozentigen wäßrigen Emulsion eines 70:30 Copolymers aus Ethylacrylat und Methylmethacrylat verbunden werden, und wobei die Überzugsmischung auf 500 Anteile von Aspirinkörnchen mit einer Maschengröße von 0,84014-0,59436 mm (20-30 Maschenzahl) aufgetragen wird, und zwar durch ein geeignetes Luftsuspensionsverfahren, so daß diskrete, überzogene Körnchen vorgesehen werden;
(2) Bereitstellung von Füllkörnchen, die pharmazeutisch inert sind und in der Größe grob mit den Aspirinkörnchen aus Teil (1) vergleichbar sind; und
(3) Komprimierung einer homogenen Mischung von 65 bis 90 Anteilen der genannten Füllkörnchen, 27 Anteilen der genannten überzogenen Aspirinkörnchen und der Gleitmittel, Zerfallsmittel und Verarbeitungshilfsstoffe, die zur Erzeugung von Tabletten erforderlich sind, die sich in weniger als 15 Minuten auflösen, die Aspirin mit einer Geschwindigkeit von 5 bis 15 mg/Stunde zuführen und die jeweils 40 bis 100 mg Aspirin aufweisen.

8. Verfahren nach Anspruch 7, wobei das Verfahren ferner folgendes umfaßt:
(4) Überzug der Tabletten mit einem in Wasser schnellöslichen Film.

9. Überzogene Aspirinkörnchen, wobei die Aspirinkörnchen mit einer Formel überzogen worden sind, die ein Überzugspolymer umfaßt, das eine durchlässige und doch unlösliche Schale vorsieht, die durch den pH-Wert nicht beeinträchtigt wird, die mit den Aspirinkörnchen nicht in chemische Reaktion tritt und die die Zerfallsgeschwindigkeit der Aspirinkörnchen begrenzt; ein wasserlösliches, hydrophiles Polymer, das die Freisetzung des Aspirins durch die durchlässige und doch unlösliche Schale moduliert; ein wasserlösliches, pharmakologisch unschädliches Salz, so daß in der Schale Kristalleinschlüsse vorgesehen werden; und einen unschädlichen, wasserunlöslichen, nicht-haftenden Überzugshilfsstoff, um die Haftung bei der Gestaltung der unlöslichen Schale zu reduzieren; und Wasser in einer solchen Menge, daß eine Formel auf Wasserbasis zum Überzug der Schale auf den Aspirinkörnchen vorgesehen wird, wobei die Bestandteile in der Formel so ausgewählt und in solchen Mengen verwendet werden, daß die Aspirinkörnchen über einen Zeitraum von fünf bis acht Stunden mit einer Geschwindigkeit von 5 bis 15 mg/Stunde freigesetzt werden.

10. Überzogene Aspirinkörnchen nach Anspruch 9, die in Tablettenform gestaltet worden sind, wobei das Überzugspolymer, das eine durchlässige und doch unlösliche Schale vorsieht, eine niedrige Glasumwandlungstemperatur aufweist, so daß die Schale verformbar ist.

11. Verwendung von Aspirinkörnchen bzw. von Aspirintabletten nach einem der Ansprüche 2-4, 9 und 10 oder gemäß einem Verfahren nach einem der Ansprüche 5-8 zur Herstellung eines Medikaments zur Behandlung bzw. zur Vermeidung von Gefäßverschlußkrankheiten bei Menschen.

## Patentansprüche (Patentansprüche für folgende(n) Vertragsstaat(en): ES, GR)

1. Verfahren zur Herstellung von Aspirinkörnchen mit einer Ordnung von fast Null und mit gesteuerter Freisetzung, wobei das Verfahren den Wasserspritzüberzug der Aspirinkörnchen mit einer Suspension vorsieht, die folgendes umfaßt: 40 bis 60 Anteile eines 70:30 Copolymers aus Ethylacrylat und Methylmethacrylat mit einem Molekulargewicht von 800.000, 10 bis 20 Anteile USP 2910 Hydroxypropylmethylcellulose, 1 bis 12 Anteile Natriumchlorid und 20 bis 45 Anteile Talk in 200 bis 900 Anteilen Wasser.

2. Verfahren nach Anspruch 1, wobei das Gesamtgewicht der auf die Aspirinkörnchen gespritzten Trockenstoffe 10 bis 35 Gewichtsprozent der Körnchen darstellt.

3. Verfahren nach Anspruch 2, wobei das Gesamtgewicht der Trockenstoffe etwa 20% des Gewichts der Aspirinkörnchen ausmacht.

4. Verfahren zur Herstellung einer niedrigdosierten, sich schnell auflösenden Aspirintablette mit gesteuerter Freisetzung, wobei das Verfahren die folgenden Schritte umfaßt:
(1) Vorbereitung überzogener Aspirinkörnchen durch Auflösen von 10 bis 20 Anteilen USP 2910 Hydroxypropylmethylcellulose mit einer Viskosität von 3 bis 15 mPa (3 bis 15 cps) in 180 Anteilen Wasser, wobei 20 bis 45 Anteile Talk USP mit einer mittleren Partikelgröße von 3 µm in einer Lösung von 1 bis 12 Anteilen Natriumchlorid in 40 bis 580 Anteilen Wasser aufgelöst werden, wobei beide mit 130 bis 200 Anteilen einer 30-prozentigen wäßrigen Emulsion eines 70:30 Copolymers aus Ethylacrylat und Methylmethacrylat verbunden werden, und wobei die Überzugsmischung auf 500 Anteile von Aspirinkörnchen mit einer Maschengröße von 0,84014-0,59436 mm (20-30 Maschenzahl) aufgetragen wird, und zwar durch ein geeignetes Luftsuspensionsverfahren, so daß diskrete, überzogene Körnchen vorgesehen werden;
(2) Bereitstellung von Füllkörnchen, die pharmazeutisch inert sind und in der Größe grob mit den Aspirinkörnchen aus Teil (1) vergleichbar sind; und
(3) Komprimierung einer homogenen Mischung von 65 bis 90 Anteilen der genannten Füllkörnchen, 27 Anteilen der genannten überzogenen Aspirinkörnchen und der Gleitmittel, Zerfallsmittel und Verarbeitungshilfsstoffe, die zur Erzeugung von Tabletten erforderlich sind, die sich in weniger als 15 Minuten auflösen, die Aspirin mit einer Geschwindigkeit von 5 bis 15 mg/Stunde zuführen und die jeweils 40 bis 100 mg Aspirin aufweisen.

5. Verfahren nach Anspruch 4, wobei das Verfahren die folgenden Schritte umfaßt:
(1) Vorbereitung überzogener Aspirinkörnchen durch Auflösen von 22 Anteilen USP 2910 Hydroxypropylmethylcellulose mit einer Viskosität von 6 mPa (6 cps) in 174 Anteilen Wasser, wobei 44 Anteile Talk USP mit einer mittleren Partikelgröße von 3 µm in einer Lösung von 4 Anteilen Natriumchlorid in 213 Anteilen Wasser aufgelöst werden, wobei beide mit 218 Anteilen einer 30-prozentigen wäßrigen Emulsion eines 70:30 Copolymers aus Ethylacrylat und Methylmethacrylat verbunden werden, und wobei die Überzugsmischung auf 580 Anteile von Aspirinkörnchen mit einer Maschengröße von 0,84014-0,59436 mm aufgetragen wird;
(2) Bereitstellung von Füllkörnchen durch Mischen von 340 Anteilen wasserhaltiger Laktose, 88 Anteilen Mikrokristallincellulose USP mit einer durchschnittlichen Partikelgröße von 50 µm, 25 Anteilen einer 11-prozentigen Mischung aus Natriumcarboxymethylcellulose in Mikrokristallincellulose und 39 Anteilen vorgelierter Stärke USP/NF in einem Fließbettgranulator und Auftragen eines Bindemittels aus 18 Anteilen USP 2910 Hydroxypropylmethylcellulose mit einer Viskosität von 15 mPa (15 cps) in 239 Anteilen Wasser mit einer Temperatur von 35 bis 50°C; und
(3) Komprimierung einer homogenen Mischung von 65 Anteilen der genannten Füllkörnchen, 27 Anteilen der genannten überzogenen Aspirinkörnchen, 4 Anteilen Natriumstärkeglycolat USP, 1 Anteil Talk USP und 2 Anteilen Stearinsäure NP in einer Standard-Tablettenpresse, so daß Tabletten erzeugt werden, die jeweils ungefähr 335 mg wiegen und 75 mg Aspirin aufweisen.

6. Verfahren nach Anspruch 4 oder 5, wobei das Verfahren ferner folgendes umfaßt:
(4) Überzug der Tabletten mit einem in Wasser schnellöslichen Film.

7. Verfahren nach Anspruch 6, mit
(4) einem Filmüberzug der Tabletten mit einer homogenen Mischung von 62 Anteilen USP 2910 Hydroxypropylmethylcellulose mit einer Viskosität von 6 mPa (6 cps), 12 Anteilen Polyethylenglycol 8000 USP/NF, 21 Anteilen Titandioxid USP und 4 Anteilen Talk USP in 900 Anteilen Wasser bei 50-65°C, so daß das Endgewicht jeder Tablette 345 mg beträgt.

8. Verfahren zur Erzeugung von Aspirinkörnchen der Ordnung von ungefähr Null und mit gesteuerter Freisetzung, wobei die Aspirinkörnchen einen Spritzüberzug mit einer Formel empfangen, die ein Überzugspolymer umfaßt, das eine durchlässige und doch unlösliche Schale vorsieht, die durch den pH-Wert nicht beeinträchtigt wird, die mit den Aspirinkörnchen nicht in chemische Reaktion tritt und die die Zerfallsgeschwindigkeit der Aspirinkörnchen begrenzt; ein wasserlösliches, hydrophiles Polymer, das die Freisetzung des Aspirins durch die durchlässige und doch unlösliche Schale moduliert; ein pharmakologisch unschädliches Salz, so daß in der Schale Kristalleinschlüsse vorgesehen werden; und einen unschädlichen, wasserunlöslichen, nicht-haftenden Überzugshilfsstoff, um die Haftung bei der Gestaltung der unlöslichen Schale zu reduzieren; und Wasser in einer solchen Menge, daß eine Formel auf Wasserbasis zum Überzug der Schale auf den Aspirinkörnchen vorgesehen wird, wobei die Bestandteile in der Formel so ausgewählt und in solchen Mengen verwendet werden, daß die Aspirinkörnchen über einen Zeitraum von fünf bis acht Stunden mit einer Geschwindigkeit von 5 bis 15 mg/Stunde freigesetzt werden.

9. Verfahren nach Anspruch 8, wobei zu den überzogenen Aspirinkörnchen Füllkörnchen gemischt werden, die pharmazeutisch inert sind und die ungefähr die gleiche Größe wie die überzogenen Aspirinkörnchen aufweisen, und wobei eine homogene Mischung der Füllkörnchen mit den überzogenen Aspirinkörnchen und anderen für die Erzeugung der Tabletten erforderlichen Gleitmittel, Zerfallshilfsstoffe und Verarbeitungshilfsstoffen, die sich in weniger als 15 Minuten auflösen, gepreßt wird.

## Revendications (Revendications pour l'(les) Etat(s) contractant(s) suivant(s): AT, BE, CH, DE, DK, FR, GB, IT, LI, NL, SE)

1. Utilisation d'une formulation à base d'eau pour recouvrir des granules d'aspirine, afin de fournir des granules recouverts qui présentent une cinétique de libération environ d'ordre zéro pour des vitesses de libération de 5 à 15 mg/hr pendant une durée s'étendant de cinq à huit heures, ladite formulation étant essentiellement constituée (a) de 40 à 60 parties d'un copolymère d'acrylate d'éthyle et de méthacrylate de méthyle 70/30, dont le poids moléculaire moyen est de 800 000 ; (b) de 10 à 20 parties d'hydroxypropylméthylcellulose de viscosité ASTM 3 à 15 mPa (3 à 15 cps) et dont la teneur en groupes hydroxypropyle est comprise entre 7 et 12 % en poids et la teneur en groupes méthoxyle est comprise entre 28 et 30 % en poids ; (c) de 1 à 12 parties de chlorure de sodium; (d) de 20 à 45 parties de talc USP ; et (e) de 200 à 900 parties d'eau.

2. Granules d'aspirine de taille de particule comprise entre 0,5 et 1,5 mm recouverts de 10 à 35 % en poids sec d'une formulation selon la revendication 1.

3. Comprimé d'aspirine faiblement dosé, à désintégration rapide, à libération contrôlée comprenant de 40 à 100 mg de granules d'aspirine recouverts d'une formulation selon la revendication 1.

4. Comprimé d'aspirine selon la revendication 3, qui comprend un ou plusieurs excipients, liants, agents de glissement, lubrifiants, diluants, plastifiants, films de revêtement, auxiliaires de formation en comprimés, activateurs de désintégration et granules de charge, de telle sorte que le comprimé se désintègre en 15 minutes et permette d'obtenir une vitesse de libération de l'aspirine de 5 à 15 mg par heure.

5. Procédé de préparation de granules d'aspirine à libération contrôlée, environ d'ordre zéro, qui comprend le revêtement par pulvérisation aqueuse de granules d'aspirine avec une suspension comprenant 40 à 60 parties d'un copolymère d'acrylate d'éthyle et de méthacrylate de méthyle 70/30, dont le poids moléculaire est de 800 000, 10 à 20 parties d'hydroxypropylméthylcellulose USP 2910, 1 à 12 parties de chlorure de sodium et 20 à 45 parties de talc dans 200 à 900 parties d'eau.

6. Procédé selon la revendication 5, dans lequel le poids total de solides secs dont on a recouvert par pulvérisation lesdits granules d'aspirine est compris entre 10 et 35 % du poids desdits granules.

7. Procédé de préparation d'un comprimé d'aspirine faiblement dosé, à désintégration rapide, à libération contrôlée, qui comprend les étapes consistant à :
(1) préparer des granules d'aspirine recouverts en dissolvant 10 à 20 parties d'hydroxypropylméthylcellulose USP 2910 de viscosité 3 à 15 mPa (3 à 15 cps) dans 90 à 180 parties d'eau, en mettant en suspension 20 à 45 parties de talc USP ayant une taille moyenne de particule de 3 µm dans une solution de 1 à 12 parties de chlorure de sodium dans 40 à 580 parties d'eau, en combinant ces deux préparations avec 130 à 200 parties d'une émulsion aqueuse à 30 % d'un copolymère d'acrylate d'éthyle et de méthacrylate de méthyle 70/30 et en appliquant le mélange de revêtement à 580 parties de granules d'aspirine de 0,84074 à 0,59536 mm d'ouverture de maille (maille 20 à 30) par un procédé de revêtement par suspension à l'air adéquat, afin d'obtenir des granules revêtus discrets;
(2) fournir des granules de charge inertes du point de vue pharmaceutique et de taille à peu près comparable à celle des granules d'aspirine de la partie (1); et
(3) comprimer un mélange homogène de 65 à 90 parties desdits granules de charge, 27 parties desdits granules d'aspirine recouverts, et d'autres substances telles qu'agents de glissement, désintégrants et auxiliares de fabrication qui peuvent être nécessaires à la production de comprimés qui se désintégrent en moins de 15 minutes, qui délivrent l'aspirine à une vitesse de 5 à 15 mg/hr et qui contiennent de 40 à 100 mg d'aspirine chacun.

8. Procédé selon la revendication 7, qui comprend l'étape consistant à (4) recouvrir d'un film lesdits comprimés avec un film rapidement soluble dans l'eau.

9. Granules d'aspirine recouverts, lesquels granules ont été recouverts d'une formulation comprenant un polymère de revêtement fournissant une enveloppe perméable mais insoluble, qui n'est pas affectée par le pH, n'interagit pas chimiquement avec les granules d'aspirine, et limite la vitesse de dissolution des granules d'aspirine ; un polymère hydrophile hydrosoluble conçu pour moduler la libération de l'aspirine grâce à l'enveloppe perméable mais insoluble ; un sel hydrosoluble inoffensif du point de vue pharmacologique apportant des inclusions cristallines dans l'enveloppe ; et un auxiliaire de revêtement anti-adhésif hydrosoluble inoffensif permettant de réduire le pouvoir collant lors de la formation de l'enveloppe insoluble, et de l'eau en une quantité permettant de fournir une formulation à base aqueuse pour le revêtement de ladite enveloppe sur lesdits granules d'aspirine, les composants de ladite formulation étant choisis et utilisés en quantités permettant la libération des granules d'aspirine à une vitesse comprise entre 5 et 15 mg/hr pendant une durée comprise entre cinq et huit heures.

10. Granules d'aspirine recouverts selon la revendication 9, formés en comprimés, dans lequel le polymère de revêtement, qui fournit l'enveloppe perméable mais insoluble, a une température de transition vitreuse permettant d'obtenir une déformabilité de l'enveloppe.

11. Utilisation de granules d'aspirine ou de comprimés d'aspirine selon l'une quelconque des revendications 2 à 4, 9 et 10, ou préparés par un procédé selon l'une quelconque des revendication 5 à 8, pour préparer un médicament pour le traitement ou la prévention de maladie vasculaire occlusive chez l'homme.

## Revendications (Revendications pour l'(les) Etat(s) contractant(s) suivant(s): ES, GR)

1. Procédé de préparation de granules d'aspirine à libération contrôlée, environ d'ordre zéro, qui comprend le revêtement par pulvérisation aqueuse de granules d'aspirine avec une suspension de 40 à 60 parties d'un copolymère d'acrylate d'éthyle et de méthacrylate de méthyle 70/30 dont le poids moléculaire est de 800 000, de 10 à 20 parties d'hydroxypropylméthylcellulose USP 2910, de 1 à 12 parties de chlorure de sodium et de 20 à 45 parties dans 200 à 900 parties en poids d'eau.

2. Procédé selon la revendication 1, dans lequel le poids total des solides secs dont on a recouvert par pulvérisation lesdits granules d'aspirine est compris entre 10 et 35 % du poids desdits granules.

3. Procédé selon la revendication 2, dans lequel le poids total des solides secs constitue 20 % du poids desdits granules d'aspirine.

4. Procédé de préparation d'un comprimé d'aspirine faiblement dosé, à désintégration rapide, à libération contrôlée, qui comprend les étapes consistant à :
(1) préparer des granules d'aspirine recouverts en dissolvant 10 à 20 parties d'hydroxypropylméthylcellulose USP 2910 de viscosité 3 à 15 mPa (3 à 15 cps) dans 90 à 180 parties d'eau, en mettant en suspension 20 à 45 parties de talc USP ayant une taille de particule moyenne de 3 µm dans une solution de 1 à 12 parties de chlorure de sodium dans 40 à 580 parties d'eau, en combinant ces deux préparations avec 130 à 200 parties d'une émulsion aqueuse à 30 % d'un copolymère d'acrylate d'éthyle et de méthacrylate de méthyle 70/30 et en appliquant le mélange de revêtement à 580 parties de granules d'aspirine de 0,84074 à 0,59536 mm d'ouverture de maille (maille 20 à 30) par un procédé de revêtement par suspension à l'air adéquat, afin d'obtenir des granules revêtus discrets;
(2) fournir des granules de charge qui sont inertes du point de vue pharmaceutique et de taille à peu près comparable à celle des granules d'aspirine de la partie (1); et
(3) comprimer un mélange homogène de 65 à 90 parties desdits granules de charge, 27 parties desdits granules d'aspirine recouverts, et d'autres agents de glissement, désintégrants et auxiliaires de fabrication qui peuvent être nécessaires à la production de comprimés qui se désintégrent en moins de 15 minutes, qui délivrent l'aspirine à une vitesse de 5 à 15 mg/hr et qui contiennent de 40 à 100 mg d'aspirine chacun.

5. Procédé selon la revendication 4, qui comprend les étapes consistant à :
(1) préparer des granules d'aspirine recouverts en dissolvant 22 parties d'hydroxypropylméthylcellulose USP 2910 de viscosité 6 mPa (6 cps) dans 174 parties d'eau, en mettant en suspension 44 parties de talc USP ayant une taille de particule moyenne de 3 µm dans une solution de 4 parties de chlorure de sodium dans 213 parties d'eau, en combinant ces deux préparations avec 218 parties d'une émulsion aqueuse à 30 % d'un copolymère d'acrylate d'éthyle et de méthacrylate de méthyle 70/30, et en appliquant le mélange de revêtement à 580 parties de granules d'aspirine de 0,84074 à 0,59536 mm d'ouverture de maille (maille 20 à 30);
(2) préparer des granules de charge en mélangeant 340 parties de lactose aqueux USP, 88 parties de cellulose microcristalline USP de taille moyenne de particule de 50 µm, 25 parties d'un mélange de 11 % de carboxyméthylcellulose dans de la cellulose microcristalline, et 39 parties d'amidon prégélatinisé USP/NF dans un granulateur en lit fluidisé, et en appliquant un liant constitué de 18 parties d'hydroxypropylméthylcellulose d'une viscosité de 15 mPa (15 cps) dans 239 parties d'eau à une température comprise entre 35 et 50°C ; et
(3) comprimer un mélange homogène de 65 parties desdits granules de charge, 27 parties desdits granules d'aspirine recouverts, 4 parties de glycolate d'amidon sodique USP, 1 partie de talc USP et 2 parties d'acide stéarique NE dans une presse à comprimés standard pour produire des comprimés pesant environ 335 mg et contenant de 75 mg d'aspirine chacun.

6. Procédé selon la revendication 4 ou la revendication 5, qui comprend l'étape consistant à (4) recouvrir d'un film lesdits comprimés avec un film rapidement soluble dans l'eau.

7. Procédé selon la revendication 6, dans lequel l'étape (4) comprend le revêtement par un film desdits comprimés avec un mélange homogène de 62 parties d'hydroxypropylméthylcellulose USP 2910 d'une viscosité de 6 mPa (6 cps), 12 parties de polyéthylène glycol 8000 USP/NF, 21 parties de dioxyde de titane USP, et 4 parties de talc USP dans 900 parties d'eau à 50-65°C de telle sorte que le poids final de chacun des comprimés soit de 345 mg.

8. Procédé de préparation de granules d'aspirine à libération contrôlé, environ de l'ordre de zéro, qui comprend le revêtement des granules d'aspirine par pulvérisation avec une formulation comprenant un polymère de revêtement fournissant une enveloppe perméable mais insoluble qui n'est pas affectée par le pH, n'interagit pas chimiquement avec les granules d'aspirine, et limite la vitesse de dissolution des granules d'aspirine ; un polymère hydrophile hydrosoluble conçu pour moduler la libération de l'aspirine grâce à l'enveloppe perméable mais insoluble ; un sel hydrosoluble inoffensif du point de vue pharmacologique apportant des inclusions cristallines dans l'enveloppe ; et un auxiliaire de revêtement inoffensif hydrosoluble anti-adhésif permettant de réduire le pouvoir collant lors de la formation de l'enveloppe insoluble, et de l'eau en une quantité permettant de fournir une formulation à base aqueuse pour recouvrir lesdits granules d'aspirine de ladite enveloppe, les composants de ladite formulation étant choisis et utilisés en quantités permettant la libération des granules d'aspirine à une vitesse comprise entre 5 et 15 mg/hr pendant une durée comprise entre cinq et huit heures.

9. Procédé selon la revendication 8, qui comprend le mélange des granules d'aspirine recouverts avec des granules de charge qui sont inertes du point de vue pharmaceutique et de taille à peu près comparable à celle des granules d'aspirine recouverts, et la compression d'un mélange homogène des granules de charge avec les granules d'aspirine recouverts et d'autres substances telles qu'agents de glissement, désintégrants et auxiliaires de fabrication qui peuvent être nécessaires pour produire des comprimés qui se désintègrent en moins de 15 minutes
